# EUROPEAN PATENT SPECIFICATION

(11) **EP 0 907 373 B1**
(45) Date of publication and mention of the grant of the patent: **07.07.2004**
(21) Application number: 97923002.6
(22) Date of filing: 07.05.1997
(51) Int. Cl.: A61K 38/21

(54) **USE OF SYNERGISTIC COMPOSITIONS OF IL-12 AND IFN-ALPHA FOR THE TREATMENT OF INFECTIOUS DISEASES**
VERWENDUNG VON SYNERGISTISCHEN ZUSAMMENSETZUNGEN VON IL-12 UND IFN-ALPHA FÜR DIE BEHANDLUNG VON INFEKTIÖSEN KRANKHEITEN
UTILISATION DE COMPOSITIONS SYNERGISTIQUES D'IL-12 ET IFN-ALPHA POUR LE TRAITEMENT DE MALADIES INFECTIEUSES

(30) Priority: 13.05.1996 GB 9609932
(43) Date of publication of application: 14.04.1999
(73) Proprietor: F. HOFFMANN-LA ROCHE AG, 4070 Basel (CH)
(72) Inventor: ALBER, Gottfried, D-04155 Leipzig (DE); CARR, Jacqueline, Anne, Ware, Hertfordshire SG12 OEP (GB); MATTNER, Frank, Albert, I-20122 Mailand (IT); MULQUEEN, Michael, John, Rochford, Essex SS4 1RJ (GB); PALMER, Kathrin, CH-4142 Münchenstein (CH); ROGERSON, Jane, Andre, Louise, St. Albans, Hertfordshire AL4 9LZ (GB)
(74) Representative: Schreiner, Siegfried, Dr.
(86) International application number: PCT/EP1997/002336
(87) International publication number: WO 1997/042969

(56) References cited:
- CA-A- 2 180 193
- BIOMEDICINE & PHARMACOTHERAPY, vol. 48, no. 2, 1994, pages 73-77, XP002038147 LACHGAR A. ET AL.: "Involvment of alpha-interferon in HIV-1 induced immunosuppression. A potential target for AIDS prophylaxis and treatment"
- JOURNAL OF IMMUNOLOGY, vol. 152, no. 3, 1 February 1994, BALTIMORE US, pages 1253-1264, XP002038148 ORANGE J.S. ET AL.: "Effects of IL-12 on the Response and Susceptibility to Experimental Viral Infections" cited in the application
- JOURNAL OF IMMUNOLOGY, vol. 153, no. 4, 15 August 1994, BALTIMORE US, pages 1697-1706, XP002038149 NASTALA C.L. ET AL.: "Recombinant IL-12 Administration Induces Tumor Regression in Association with IFN-gamma Production" cited in the application
- ANNALS OF THE NEW YORK ACADEMAY OF SCIENCES, vol. 795, 31 October 1996, pages 354-356, XP002038150 ROGERSON J.A.L. ET AL.: "Co-therapy with Suboptimal Doses of Interleukin-12 and Interferon-alpha promotes synergistic protection against Herpes Virus Infection"
- DATABASE BIOSIS BIOSCIENCES INFORMATION SERVICE, PHILADELPHIA, PA, US CARR J A ET AL: "Dual therapy with IL - 12 and IFN - alpha provides a novel approach to the management of chronic viral disease." XP002038151 & MEETING OF THE INTERNATIONAL SOCIETY FOR ANTIVIRAL RESEARCH AND THE TENTH INTERNATIONAL CONFERENCE ON ANTIVIRAL RESEARCH, ATLANTA, GEORGIA, USA, APRIL 6-11, 1997. ANTIVIRAL RESEARCH 34 (2). 1997. A65,

## Description

The present invention relates to the field of prevention and treatment of infectious diseases using a synergistic combination of Interleukin-12 (IL-12) and Interferon-α (IFNα). This combination is especially useful for the prophylaxis and treatment of chronic infectious diseases, e.g. viral infections, intracellular bacterial infections and parasite infections.

Infectious diseases are major causes of adult morbidity and mortality (Marglois (1993) J. Infect. Dis. 168, 9-14). For instance, viral induced liver disease has been linked to the induction of cirrhosis and primary liver carcinomas which may account for the related 1.5 million deaths per year. Present estimates suggest over 300 million people worldwide may be chronic carriers of Hepatitis B infection, with in excess of 50 million new cases per annum (Finter et al (1991) Drugs 42, 749-765). Although less common, chronic Hepatitis C infection has also been implicated in subsequent development of liver cirrhosis and hepatocellular carcinoma (3), and may be the major cause of liver disease in Western nations (Scrip 2170 (1996) p. 20). These virus infections therefore represent areas of major medical need.

The persistence of these infections has been linked to the induction of T-cell tolerance and failure to clear the virus (Reis & Rouse (1993) Immunol. Today 14, 333-335). Current therapy with type I Interferons, notably IFNα, have been successful in promoting viral clearance, as measured by the disappearance of viral RNA from the serum, and the seroconversion of patients. However, sustained benefit is usually seen in only a proportion of patients, with 25-30% of Hepatitis B patients (Finter et al., loc. cit.) showing long-term responses. Responsiveness by Hepatitis C patients is variable, and may depend on infecting viral genotype (Clarysse et al. (1995) Netherlands J. Med. 47, 265-271). Side-effects of type I IFN therapy, including ,flu-like' symptoms, fatigue, weight loss and reduced platelet counts, limit the maximum dose routinely used in the clinic, and may account for the relatively low response rates seen. Thus, therapies which can complement the actions of type I IFN s have the potential to increase the response rate and improve the clinical benefit of immunotherapy for chronic hepatitis virus infection.

Interleukin 12 (IL-12), formerly called natural killer cell stimulatory factor (Kobayashi et al. (1989) J. Exp. Med. 170, 827-845) and cytotoxic lymphocyte maturation factor (Stem et al. (1990) Proc. Natl. Acad. Sci. USA 87, 6808-6812), has potent anti-tumor and antimetastatic activity in several murine tumor models (Brunda et al. (1993) J. Exp. Med. 178, 1223-1230; Nastala et al. (1994) J. Immunol. 153, 1697-1706). Although the mechanism through which IL-12 exerts its anti-tumor effects is not completely understood, it has been shown that IL-12 induces a variety of biological effects on natural killer and T cells *in vitro* (Manetti et al. (1994) J. Exp. Med. 179, 1273-1283; Wu et al. (1993) J. Immunol. 151, 1938-1949; Tripp et al. (1993) Proc. Natl. Acad. Sci. USA 90, 3725-3729; Seder et al. (1993) Proc. Natl. Acad. Sci. USA 90, 10188-10192; Bloom et al. (1994) J. Immunol. 152, 4242-4254; Cesano et al. (1993) J. Immunol. 151, 2943-2957; Chan et al. (1992) J. Immunol. 148, 92-98). Activation of cytotoxic T lymphocytes by IL-12 is considered crucial in its anti-tumor activity (Brunda et al. (1993) J. Exp. Med. 178, 1223-1230). The IL-12 anti-tumor effect is partially maintained in severe combined immune deficient (SCID) and nude mice, both of which are T cell-deficient, and in CD8⁺-depleted euthymic mice (Brunda et al. (1993) J. Exp. Med. 178, 1223-1230; O'Toole et al. (1993) J. Immunol. 150, 294A). These results demonstrate that IL-12 has potent *in vivo* antitumor and antimethastatic effects against murine tumors and demonstrate as well the critical role of CD8⁺ T cells in mediating the antitumor effects against subcutaneous tumors.

Interferons (IFNs) are naturally occurring proteins which have antiviral, antiproliferative and immunoregulatory activity. Four distinct classes of interferons are known to exist in humans (Pestka et al. (1987) Ann. Rev. Biochem. 56, 727-777 and Emanuel & Pestka (1993) J. Biol. Chem. 268, 12565-12569). The IFNα family represents the predominant class of IFNs produced by stimulated peripheral blood leukocytes (Pestka et al., loc. cit.; Havell et al. (1975) Proc. Natl. Acad. Sci. USA 72, 2185-2187; Cavalieri et al. (1977) Proc. Natl. Acad. Sci. USA 74, 3287-3291), and lymphoblastoid and myeloblastoid cell lines (Familletti et al. (1981) Antimicrob. Agents. Chemother. 20, 5-9). The antiviral effect of IFNα is achieved not by a direct influence on the viruses themselves, but by an activity on their target cells in the sense of a protection against the virus infection. The interferons can exert effects on cancer tumors and can influence the immune system of the body on that, for example, they activate macrophages and NK cells and intensify the expression of various immunologically significant constituents of the cell membrane. Details of the preparation of interferon-cDNA and the direct expression thereof, especially in E. coli, have been the subject of many publications. Thus, for example, the preparation of recombinant interferons is known, for example, from Nature 295 (1982), 503-508, Nature 284 (1980), 316-320, Nature 290 (1981), 20-26, Nucleic Acids Res. 8 (1980), 4057-4074, as well as from European Patents Nos. 32134, 43980 and 211148.

IFNα has proven to be effective in the treatment of viral infections, e.g. both Hepatitis B and Hepatitis C virus (HBV, HCV) infections, however a significant number of patients do not respond to this cytokine. The ability of IL-12 to promote both Th1 maturation and the enhancement of CTL and NK activity is likely to be critical for its ability to protect against disease in mouse models of viral infections (Orange et al. (1994) J. Immunol. 152, 1253 - 1264).

The present invention relates to the field of prevention and treatment of infectious diseases using IL-12 in a synergistic combination with IFNα. Surprisingly, sub-optimal doses of IL-12 and IFNα promote effective protection *in vitro* and *in vivo* against infectious diseases, especially against viral and parasite infections and intracellular bacterial infections.

In accordance with the present invention, a synergistic combination of IL-12 and IFNα together with a pharmaceutically acceptable carrier is provided which is effective in treatment and prophylaxis of infectious diseases, preferably chronic infectious diseases and more preferably viral infections, e.g. Herpes (HSV), HIV, Hepatitis B, Hepatitis C, etc., intracellular bacterial infections, e.g. tuberculosis, salmonellosis, listeriosis, etc., and parasite infections, e.g. malaria, leishmaniasis, schistosomiasis. These compositions are characterised by the synergistic interaction of IL-12 and IFNα. They are easily administered by different routes including parenteral and can be given in dosages that are safe and sufficient to treat or prevent infectious diseases. The above pharmaceutical compositions may contain additional compounds useful for the treatment of infectious diseases. The present invention also provides the use of the above compounds for the manufacture of medicaments for the treatment and prophylaxis of infectious diseases mentioned above.

### Brief description of the drawings

Figure 1: *In vitro* differentiation of murine T helper lymphocytes into mature Th1 or Th2 cells. CD4+ T lymphocytes were purified from mouse spleen. Cells were activated with immobilised anti-CD3 antibodies. After 5 days of incubation with IL-12, IL-4 and/or IFNα the supernatants were analysed for IFNγ and IL-10 by ELISA.
Figure 2: IFNγ production by helper T lymphocytes differentiated with IL-12 and/or IFNα. Purified CD4+ splenocytes were activated with anti-CD3. The cells were incubated for 5 days in medium containing IL-12 and/or IFNα.
Figure 3: IL-10 production by helper T lymphocytes differentiated with IL-12 and/or IFNα. Purified CD4⁺ splenocytes were activated with anti-CD3. The cells were incubated for 5 days in medium containing IL-12 and/or IFNα. IL-10 was determined by ELISA.
Figure 4: IL-12 and IFNα co-administration protects against systemic HSV infection: BALB/c mice (15 per group) were infected with HSV-2 after treatment with IL-12, IFNα or a combination of these two cytokines, as detailed in methods. Mortality was assessed daily up until day 20 p.i. and compared to infected, untreated control animals.
Figure 5: Effect of IFNγ depletion on IL-12 + IFNα protection against HSV infection: Anti-interferon-γ antibody (XMG 1.2, 100 µg i.p.) was administered on days -3, -1, 0, 2 and 4 relative to the time of infection with HSV-2. Animals were treated with IL-12 and IFN-α as detailed in the Examples.
Figure 6: IFNγ production by spleen cell cultures stimulated with HSV-2 antigen: IFN-γ production from UV inactivated HSV-2 stimulated spleen cells, cultured on day 10 post infection.

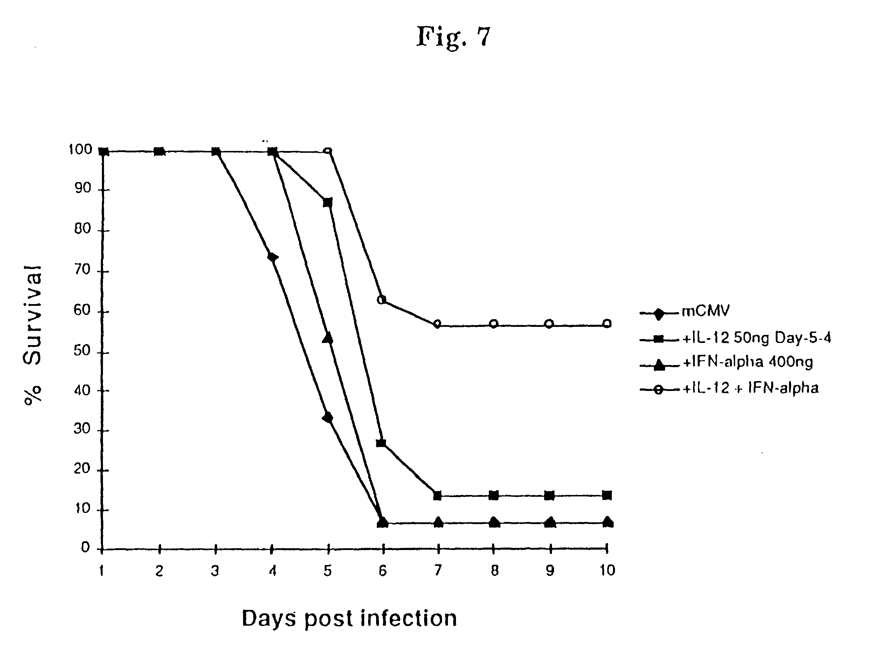

Figure 7: IL-12 + IFNα co-administration protects against systemic mCMV infection: BALB/c mice (15 per group) were infected with mCMV after treatment with IL-12, IFNα or a combination of these two cytokines, as detailed in methods. Mortality was assessed daily up until day 14 p.i.

The present invention provides the use of a synergistic combination of IL-12 and IFNα for the preparation of medicaments for the treatment of infectious diseases, especially chronic infectious diseases. These infectious diseases are caused by transfer of viral, bacterial parasite and of other microorganisms. A particular use of the synergistic combination of IL-12 and IFNα according to the present invention is the preparation of medicaments for the prophylaxis and treatment of viral diseases, preferably chronic viral infections, e.g. hepatitis, herpes, papilloma, or human immunodeficiency virus infections. A further embodiment of the present invention comprises the use of the above compounds for the prophylaxis and treatment of bacterial infectious diseases, preferably intracellular bacterial infectious diseases, e.g. tuberculosis, salmonellosis or listerosis. The present invention relates also to the use of the above compounds for the treatment of parasite infections. Parasite infectious diseases comprise infectious diseases like malaria, leishmaniosis or schistosomiasis.

In addition, the present invention also relates to the corresponding pharmaceutical compositions useful for the treatment of the above diseases. The pharmaceutical compositions are characterised in that they contain a synergistic combination of IL-12, IFNα and a pharmaceutically acceptable carrier. These compositions may contain one or more additional compounds useful for the prophylaxis and treatment of infectious diseases.

The results of the present invention indicate that suboptimal doses of IL-12 and IFNα can synergise to provide protection against infectious diseases, and this is mediated at least in part by IFNγ. Surprisingly, data from *in vitro* experimental work show increased production of IFNγ from spleen cells of IL-12/IFNα treated animals. Thus, an enhanced Th1 response to infectious diseases underlines the beneficial effects of IFNα/IL-12 combination therapy.

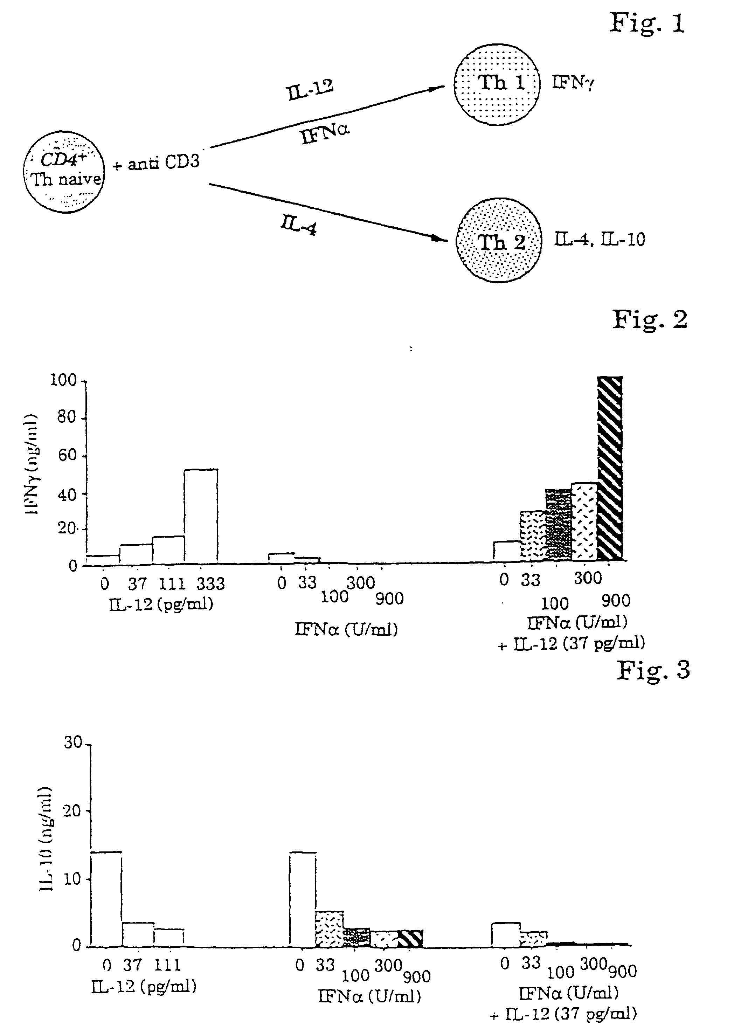

An *in vitro* Th1/Th2 differentiation system has been established to analyse the role of IFNα and IL-12. Figure 1 depicts the principal characteristics of the *in vitro* differentiation of T helper lymphocytes into mature Th1 or Th2 cells. The experiment shown in Figure 2 demonstrates synergism of IFNα with suboptimal doses of IL-12 in the induction of IFNγ as a measure of Th1 cell activity. This is associated with inhibition of IL-10 production (a Th2 lymphokine) by IFNα (Figure 3).

These data are further supported in models of herpes virus infection, using Herpes simplex virus type 2 (HSV-2) and murine cytomegalovirus (mCMV).

### Systemic infection with HSV-2:

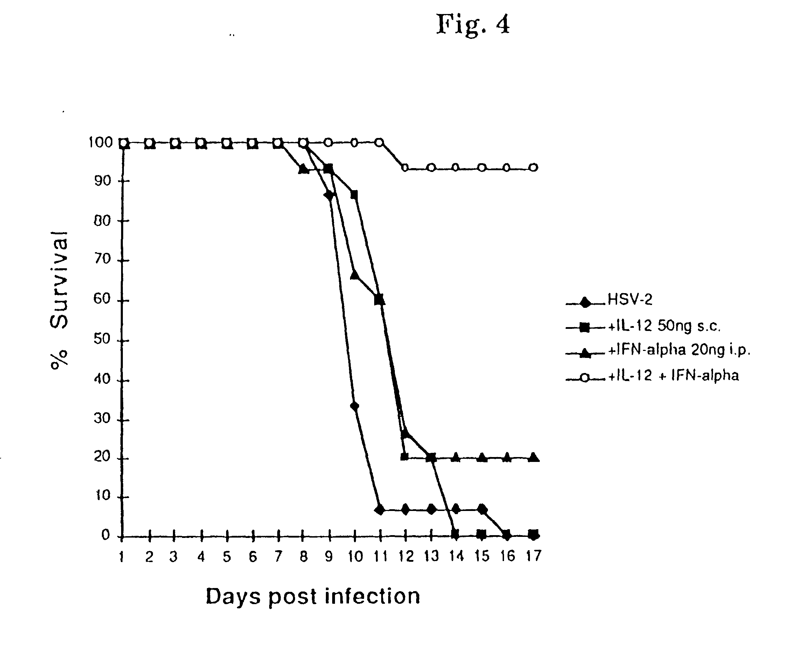

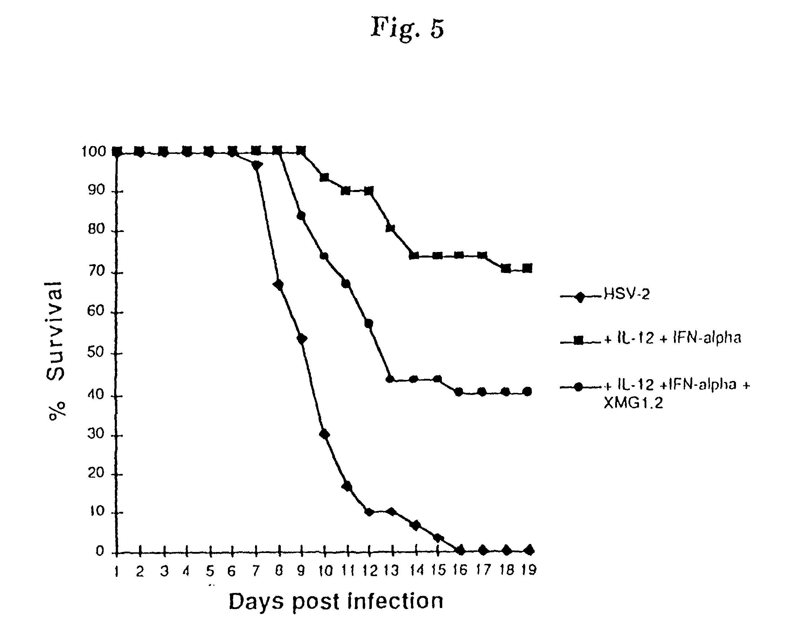

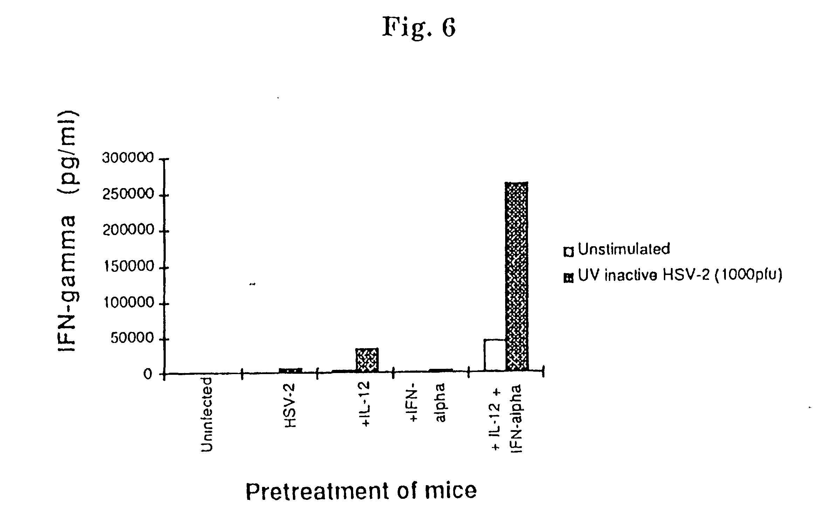

Following an i.p. infection, HSV-2 distributes to multiple sites, particularly the peripheral and central nervous system. Subsequent replication within the CNS produces a lethal encephalitis. IL-12 and IFNα have both been shown to provide dose-dependent protection from the lethal effects of systemic HSV-2 infection . In Example 2 50 ng IL-12 s.c. per mouse and 20 ng IFNα i.p. were used per mouse, as sub-optimal doses of cytokines still allowing 100% and 80% HSV induced mortality respectively (Figure 4). When these sub-therapeutic doses of both IL-12 and IFNα were administered together a highly significant improvement on survival on day 20 p.i. (p<0.01) was seen with a reduction to 22% ± 9 mortality (Table 1), compared to 94% ± 4 in control groups. Supernatants taken from UV inactivated HSV-2 stimulated spleen cell cultures showed greater quantities of IFNγ in the cultures taken from animals receiving co-therapy, than in those undergoing mono-therapy with either cytokine alone (Figure 6). The highly increased survival seen with co-therapy can be partially reversed by prior depletion of the animals of IFNγ using the IFNγ neutralising antibody XMG 1.2. Survival is reduced by 30% (p<0.06), compared to co-therapy not receiving IFNγ depletion (Figure 5). This indicated that IFNγ is only partially responsible for the protection afforded by co-therapy.

**Table 1:**

| **Frequency of Survival of Mice with HSV-2 (333) Systemic Infection** | | | | |
|---|---|---|---|---|
| **GROUP** | **DOSE** | **% MORTALITY*** | | |
| | **(DAYS 0 -** 4) | **DAY 10** | **DAY 20** | **P Value** |
| Control | PBS/BSA | 20 ± 8 | 94 ± 4 | |
| IL-12 s.c. | 50ng | 36 ± 13 | 88 ± 5 | N.S. |
| IFNα i.p. | 20ng | 18 ± 8 | 74 ± 3 | N.S. |
| IL-12 s.c. | 50ng | | | |
| + IFNα | +20ng | 4±2 | 22 ± 9 | <0.01 |
| i.p. | | | | |

| | | | | |
|---|---|---|---|---|
| * Mean ± s.e.m. of three experiments | | | | |

### Zosteriform infection with HSV-2:

Co-administration of IL-12 and IFNα enhanced survival rates in the more severe model of zosteriform HSV-2 infection. Inoculation of HSV-2 at the skin surface results in infection of sensory neurons, retrograde axonal transport of virus to sensory ganglia and replication at this site. Subsequently, virus emerges at skin sites innervated by axons from the infected ganglia to produce the characteristic zoster lesion. Animals also show progressive infection of the CNS with development of lethal encephalitis. Survival of this infection was significantly increased using IL-12 and IFNα co-therapy (Table 2) by 40% compared to untreated virus infected mice, or mice treated with mono-therapy alone (p<0.01). Co-therapy also reduced the severity of zoster lesion development, although this effect was more variable (significant reduction in 2 out of 3 trials).

### Systemic infection with mCMV;

Murine CMV infection causes pathological changes in a number of tissues throughout the body, but the major site of viral replication is the acinar cells of the salivary glands. Death is thought to occur due to organ tissue damage and the immune mediated pathology associated with it. In this lethal mCMV infection, IL-12 and IFNα dosed in combination gave greater survival rates than those groups treated with monotherapy alone. Dosing with IL-12 alone at 5 - 50 ng per mouse i.p. on days -2 and -1 gave 100% protection from lethal mCMV infection, while dosing after infection did not alter disease pathology. In order to derive a sub-optimal dosing regime for IL-12, the time of infection was delayed by 2, 4 or 6 days relative to the time of IL-12 therapy. IFNα dosed therapeutically gave 20-30% protection against mCMV induced mortality, with no clear dose-related effect. Preliminary experiments indicated 400 ng IFNα i.p. per mouse was a sub-optimal dose. Co-administration of this dose of IFNα with IL-12 therapy gave enhanced survival over controls receiving virus infection or mono-therapy alone (Table 3). These results mirror the NK activity measured using spleen cells in a ⁵¹Cr-release assay, from animals treated with the same regime of IL-12. NK activity was maximal 2 days after IL-12 treatment, and decreased in a time dependent manner thereafter (data not shown). This suggests that NK activity has a role in the IL-12-mediated survival seen in this model.

**Table 2 :**

| **Frequency of Survival of Mice with HSV-2 (333) Zosteriform Infection** | | | | |
|---|---|---|---|---|
| GROUP | DOSE | % MORTALITY* | | |
| | | DAY 7 | DAY 14 | P Value |
| Control | PBS/BSA | 3 ± 3 | 80 ± 13 | |
| IL-12 sc | 50ng | 0 | 67 ± 16 | N.S. |
| IFNα ip | 20ng | 0 | 78 ± 4 | N.S. |
| IL-12 sc | 50ng | | | |
| +IFNα ip | +20ng | 3 ± 3 | 35 ± 6 | <0.01 |

| | | | | |
|---|---|---|---|---|
| * Mean ± s.e.m. of two experiments | | | | |

**Table 3 :**

| **Frequency of Survival for Mice Infected with Murine CMV** | | | |
|---|---|---|---|
| GROUP | DOSING WINDOW | % MORTALITY* | |
| | | DAY 5 | DAY 10 |
| Control | | 78 ± 8 | 91 ± 6 |
| IL-12 | DAYS -6 -5 | 83 ± 3 | 87 ± 0 |
| 50ng ip | DAYS -5 -4 | 13 ± 0 | 87 ± 0 |
| | DAYS -4 -3 | 30 ± 19 | 80 ± 16 |
| | DAYS -2 -1 | 0 | 0 |
| IFNα 400ng ip | DAYS 0 to +4 | 51 ± 4 | 67 ±14 |
| IL-12 | DAYS -6 -5 | 50 ± 3 | 77 ± 14 |
| + IFNα | 0 to +4 | | |
| | DAYS -5 -4 | 0 | 44 ± 0 |
| | 0 to +4 | | |
| | DAYS -4 -3 | 0 | 27 ± 0 |
| | 0 to +4 | | |

| | | | |
|---|---|---|---|
| * Mean ± s.e.m. of two experiments | | | |

In summary, the results suggest that sub-optimal doses of IL-12 and IFNα together promoted effective protection *in vitro* and *in vivo* against infectious diseases. The studies indicate that enhanced survival may be correlated with an increase in Th1 induction, since spleen cells given co-therapy produced high levels of IFNγ, following *in vitro* stimulation.

The central role of IFNγ has also been shown in defense against intracellular bacteria (Listeria monocytogenes). Anti-IFNγ treatment prior to infection increases susceptibility, whereas recombinant IFNγ increases resistance. One major function of IFNγ is the activation of macrophages for their microzidal activities, e.g. production of reactive oxidative intermediates. In addition, several findings have demonstrated the importance of IFNγ for the elimination of intracellular parasites (L. major) by infected hosts. The destruction of parasites by murine macrophages was shown to result from the IFNγ-induced production of nitric oxide by these cells.

Especially, IL-12 and IFN-α in a synergistic combination provide a clear and novel advantage over monotherapy in the management of viral diseases, as shown with models of experimental herpes virus infection.

The synergistic combination regime afforded marked protection against an otherwise lethal viral (here: HSV-2) systemic infection, at dose levels which given individually were only marginally efficacious. Combination therapy also significantly reduced mortality associated with a lethal HSV-2 zosteriform infection, and importantly, has been shown to reduce lesion severity in this model of infection. This latter observation provides an indication that combination therapy would be effective in limiting clinical manifestations of viral disease. IL-12 and IFN-α in a synergistic combination also markedly improved survival rate of an otherwise lethal mCMV infection, which previously we had been unable to influence with the same treatments used individually.

In the Examples, IL-12 was administered prophylactically, that is, at a time before mice had been exposed to viral antigen. Thus it seemed likely that the contribution of IL-12 to the dramatic effect of combination therapy would be effected via non-specific mechanisms, particularly by induction of IFNγ. The data demonstrated that a substantial part of the protection afforded by co-therapy was due to induction of IFNγ. The synergistic effect of IL-12/IFN-α in combination on survival seen *in vivo* can be correlated with the antigen specific induction of IFNγ production by spleen cells *in vitro*.

Both IL-12 and IFNα are naturally occurring molecules which exert influence on host immune responses, and both cytokines have potential in the management of virus disease. As far as is known, the modes of action of IL-12 and IFNα are quite different, although recent evidence suggests that a pathway may exist by which IFNα alters expression levels of IL-12. The consequence of induction of either cytokine (or both) is to provide an immunological environment hostile to continuing viral infection.

### IL-12 exerts antiviral effects by influencing immune status.

Specifically, IL-12 effects induction of IFNγ, enhancement of NK cell lysis of virus infected cells, and induction of a Th1-type response. A consequence of development of a cellular response of Th1 phenotype is that CD8⁺ T cell cytotoxicity to virus-infected cells is enhanced, and an immunoglobulin class switch to IgG2a occurs, with inhibition of IgE synthesis. Thus IL-12 optimises the environment for effective viral clearance.

IFNα, in contrast to IL-12, is not thought to work primarily via immunomodulation, but rather induces a general antiviral state in an infected cell (Gresser et al. (1976) J. Exp. Med. 144, 1316-1323). The antiviral state involves induction of cellular mechanisms which target a range of stages in a viral replicative cycle. IFNα, activates Interferon Stimulated Response Elements (ISRE) in the cell nucleus, causing production of a number of proteins. These include the Mx protein with its specific antiviral function (Weitz et al. (1986), J. Int. Res. 9, 679-689), a protein kinase activated by double stranded RNA to phosphorylate eukaryotic initiation factor 2, in turn blocking translation and therefore virus assembly (Hovanessian (1989) J. Interferon Res. 9, 641-647), and 2',5-oligoadenylate synthetase which is involved in degradation of newly formed virus components (Pestka et al., loc. cit.) .

Combining IFN-α therapy with IL-12 would benefit patients in a dual manner, firstly the central role of IL-12 in directing immune responses may break tolerance to an infectious antigen, and secondly, the effect of IL-12 on cytotoxic T cell responses would help to ensure this response was sufficient to facilitate total clearance of the infectious agent. This cytokine combination approach to management of infectious diseases has the potential to impact on an area of significant medical need.

In summary, the results suggest that sub-optimal doses of IL-12 and IFNα together promoted effective protection *in vitro* and *in vivo* against infectious diseases. The studies indicate that enhanced survival may be correlated with an increase in Th1 induction, since spleen cells given co-therapy produced high levels of IFNγ, following *in vitro* stimulation.

Interleukin-12 may be prepared by methods known in the art, e.g. described in European Patent Application No. 433827, in International Patent Applications WO 9005147 and WO 9205256, in Kobayashi et al., J. Exp. Med. 170, 827-845 (1989) and Stern et al. (1990) Proc. Natl. Acad. Sci. USA 87, 6808-6812. Interleukin-12 may be produced by known conventional chemical synthesis, recombinant methods or may be purified form natural sources.

IFNα to be contained in the present composition may be those derived from any natural material (e.g., leukocytes, fibroblasts, lymphocytes) or material derived therefrom (e.g. cell lines), or those prepared with recombinant DNA technology. Details of the cloning of IFNα and the direct expression thereof, especially in E.coli, have been the subject of many publications. The preparation of recombinant IFNs is known, for example form Gray et al. (1982) Nature 295, 503- 508, Goeddel et al. (1980) Nature 284, 316- 320 and (1981, Nature 290, 20-26, and European Patent No. 174143. There are many types of IFNα such as IFNαI, IFNα2; and further their subtypes including but not limited to IFNα2A, IFNα2B, IFNα2C and IFNαII (also designated IFNα_{II} or ω-IFN).

The terms "IL-12" and "IFNα" suitable for pharmaceutical compositions also comprise polypeptides similar to those of the purified and/or recombinant protein but which modifications are naturally provided or deliberately engineered, e.g. molecules containing inversions, deletions, insertions and modifications (such as pegylated forms of IFNα and/or IL-12) as well as any hybrid or consensus IL-12 or IFNα molecules obtainable from the aforementioned molecules.

For practicing the synergistic combination therapy of this invention IL-12 is administered to the patient in association with IFNα, that is, the IFNα is administered during the same or different periods of time that the patient receives IL-12.

Pharmaceutically acceptable formulations containing synergistic combinations of IL-12 and IFNα in connection with this invention can be made using formulation methods known to those of ordinary skill in the art. These formulations can be administered by standard routes. In general, the formulations may be administered parenterally (e.g., intravenous, subcutaneous or intramuscular) with topical, transdermal, oral, or rectal routes also being contemplated. In addition, the formulations may be incorporated into biodegradable polymers allowing for sustained release of IL-12 and/or IFNα, the polymers being implanted in the vicinity of where drug delivery is desired. The biodegradable polymers and their use are described, for example, in detail in Brem et al. (1991) J. Neurosurg. 74, 441-446. The dosage of IL-12 and IFNα will depend on the condition being treated, the particular compound, and other clinical factors such as weight and condition of the human or animal and the route of administration of IL-12. It is to be understood that the present invention has application for both human and veterinary use. For parenteral administration to humans, a dosage of between approximately 1000 ng IL-12 to 10 ng/kg body weight, preferably between approximately 300 ng to 30 ng/kg 1 to 3 times a week is generally sufficient. For IFNα a dosage of between approximately 50 µg to 0.1 µg/ kg body weight, preferably between approximately 15 µg to 1 µg 1 to 3 times a week is generally sufficient. It will however be appreciated that the upper and lower limit given above can be exceeded when this is found to be indicated.

The formulations include those suitable for parenteral (including subcutaneous, intramuscular, intravenous, intradermal, intratracheal, and epidural) administration. The formulations may conveniently be presented in unit dosage form and may be prepared by conventional pharmaceutical techniques. Such techniques include the step of bringing into association IL-12 and/or IFNα and the pharmaceutical carrier(s) or excipient(s). In general, the formulations are prepared by uniformly and intimately bringing into association the IL-12 and IFNα with liquid carriers. Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, seated ampoules and vials, and may be stored in a freeze-dried (lyophilised) conditions requiring only the addition of the sterile liquid carrier, for example, water for injections, immediately prior to use.

Preferred unit dosage formulations are those containing a daily dose or unit, daily sub-dose, as herein above recited, or an appropriate fraction thereof, of the administered ingredient.

For the preparation of tablets, coated tablets, dragees or hard gelatine capsules the compounds of the present invention may be admixed with pharmaceutically inert, inorganic or organic excipients. Examples of suitable excipients for tablets, dragees or hard gelatine capsules include lactose, maize starch or derivatives thereof, talk or stearic acid or salts thereof.

Suitable excipients for use with soft gelatine capsules include for example vegetable oils, waxes, fats, semi-solid or liquid polyols etc. For the preparation of solutions and syrups, excipients which may be used include for example water, polyols, saccharose, invert sugar and glucose. For injectable solutions, excipients which may be used include for example water, alcohols, polyols, glycerine, and vegetable oils. For supopositories, and local or percutaneous application, excipients which may be used include for example natural or hardened oils, waxes, fats and semi-solid or liquid polyols. The pharmaceutical compositions may also contain preserving agents, solubilizing agents, stabilising agents, wetting agents, emulsifiers, sweeteners, colorants, odorants, salts for the variation of osmotic pressure, buffers, coating agents or antioxidants. They may also contain other therapeutically valuable agents.

The following Examples are intended to illustrate details of the invention.

### EXAMPLES

### Example 1: In Vitro maturation of Th1 cells

Experiments regarding the *in vitro* maturation of Th1 cells were done as follows:
a) Murine spleen cells:
   Spleens from 8 - 10 weeks old female C57BL7/6 mice were used to prepare a single cell suspension of spleen cells in Hank's medium + 1 % FCS. Red cells were removed with Geys solution and preactivated cells by a Percoll gradient centrifugation according to standard methods (Cambier et al. (1987) Scand. J. Immunol. 27, 59).
b) CD4⁺ purification:
   10⁷ splenocytes per ml were incubated in Hank's medium, 10 µg/ml mAb anti-IA/IE (clone M5/114, Bhattacharya et al. (1981) J. Immunol. 127, 2488) and 10 µg/ml mAB anti-CD8 (clone 53-6.7, Ledbetter et al. (1979) Immunol. Rev. 47, 63) were added. After 30 minutes incubation on ice and washing (3 x ) the cells were resuspended at 5 x 10⁶ cells/ml. 100 µl Magnetic Dyna Beads (Sheep anti Rat) were added and after 30 minutes rotation at 4°C the target cells (bound to the beads) were removed with a magnet. The purity of CD4⁺ cells was more than 90%. These cells were resuspended in complete IMDM at 10⁶ cells/ml.
c) Activation of CD4⁺ cells:
   48 well cell culture plates were coated with mAb anti-CD3 (clone 2C11, Leo et al. (1987) PNAS 84, 1374) at 5 µg/ml). The plates were washed (3 x) and 500 µl purified CD4⁺ cell suspension per well and cytokines (IL-12, IL-4, IFN‡A/D), native IFN‡) and their combinations were added. After incubation for 5 days at 37°C and 7.5% CO₂ cell culture was analysed for IFNγ and IL-10 using commercially available lymphokine specific ELISAs.

### Example 2: Virus infection

a) Mice:
   Specified pathogen-free, female BALB/c mice supplied by Harlan-Olac were used at 6 to 8 weeks of age.
b) Virus stocks:
   HSV-2 (strain 333) WT and mCMV Smith strain (ATCC No. VR-194) were grown in cell culture monolayers of Vero (ATCC No. CCL-81) and 3T3L1 (ECACC No. 86052701) cells respectively using DMEM culture medium (Gibco No. 22320-022) containing 2% FCS. Confluent cell monolayers were removed from flasks by agitation when complete cytopathic effect was observed. This was followed by freeze-thawing and sonification to release virus, before clarification by centrifugation at 3000 rpm for 15 mins. Virus titres were determined by standard plaque assay on cell monolayers. The stock of mCMV that is obtained from cell culture is non-virulent in mice at this stage, so a virulent stock was prepared by passage through mice. Briefly, the attenuated stock was injected into mice i.p. and the salivary glands were removed 10 days after infection. Pooled tissue was then homogenised to release virus and clarified by centrifugation at 1400 rpm 10 mins. Supernatant containing infectious virus were then be passaged through mice again to produce a stock of virus that could elicit 100% mortality *in vivo*.
c) HSV-2 infection of mice:
   For systemic infection animals were infected by a single i.p. injection of 10⁴ - 10⁵ pfu/ 100 µl. Animals were monitored daily for mortality until day 20 post infection (p.i.). For zosteriform infection, animals were infected on their left flank following scarification with a 25G needle and application of a 10 µl droplet containing 10⁶ pfu virus. Animals were monitored daily for zosteriform lesion development and mortality until day 14 p.i.
d) mCMV infection of mice:
   In all experiments for a lethal infection, mice were infected i.p. with 5 x 10³ pfu of salivary gland derived virus preparation. Animals were monitored daily for signs of infection and death rates were recorded until day 15 p.i.
e) Dosing with IL-12 and IFNα:
   50 ng of recombinant murine IL-12 (specific activity 2.1 x 10⁸U/mg (Hoffmann-La Roche, Nutley, NJ), was administered either by i.p. or subcutaneous (s.c.) injection, once daily in PBS + 1% w/v BSA at a volume of 5 ml per kg body weight. Therapeutic dosing commenced 6 hours after infection then daily for a further 4 days. Prophylactic therapy consisted of two daily injections prior to infection at times described in the results. Recombinant human IEFNα-A/D (specific activity 5 x 10⁷U/mg (Hoffmann-La Roche, Nutley, NJ) was administered by i.p. injection in PBS + 1% w/v BSA at a volume of 5 ml per kg body weight 2 hours prior to infection then 4, 24, 48, and 72 hours p.i.. Doses ranged from between 20 and 400 mg per day.
f) *In vivo* depletion of IFNγ:
   Rat anti-mouse IFNγ antibody, XMG 1.2 (Pestka et al. (1987) loc. cit.). This IFNγ neutralising antibody was administered i.p. at 5 ml per kg body weight, in sterile water for injection. To effect IFNγ depletion *in vivo* mice were each given a total of 5 doses of 100 µg per mouse IFNγ antibody on days -3 and -1 relative to infection, on day 0 and days 2 and 5 post infection. This regime reduced the levels of serum IFNγ produced in response to IL-12 treatment *in vivo* by approximately 95%.
g) *In vitro* IFNγ production:
   Single cell suspensions of spleens were obtained from each group on day 7 to 10 post infection, and were then cultured at 37°C in 5% CO₂ for 48 hours in complete RPMI 1640 (Gibco Cat. No. 52400-033) in the presence of 10³ pfu of UV inactivated HSV-2. Supernatants were then removed and frozen at -80°C before being assayed for IFNγ levels by ELISA using a commercially available kit (Amersham Cat No. RPN 2717).

## Claims

1. Use of a synergistic combination of Interleukin-12 (IL-12) and Interferon-alpha (IFN-α) for the manufacture of a medicament for the treatment and prophylaxis of infections.

2. Use according to claim 1 wherein the infection is a chronic infection.

3. Use according to claim 2 wherein the infection is a chronic viral infection.

4. Use according to claim 3 wherein the infection is a hepatitis, papilloma, human immunodeficiency or a herpes virus infection.

5. Use according to claims 1 or 2 wherein the infection is a bacterial infection.

6. Use according to claim 5 wherein the infection is tuberculosis, salmonellosis or listeriosis.

7. Use according to claim 1 or 2 wherein the infection is a parasitic infection.

8. Use according to claim 7 wherein the infection is malaria, leishmaniosis or schistosomiasis.

9. Interleukin-12 and Interferon-α in a synergistic combination for the treatment of diseases as mentioned in any one of claims 1 to 8.

10. Interleukin-12 and Interferon-α in a synergistic combination with one or more additional compounds for the treatment of diseases as mentioned in any of claims 1 to 8.

11. A pharmaceutical composition containing a synergistic combination of Interleukin-12 and Interferon-α together with a pharmaceutically acceptable carrier.

12. The pharmaceutical composition of claim 11 containing one or more additional compounds useful for the treatment of infectious diseases.

## Patentansprüche

1. Verwendung einer synergistischen Kombination von Interleukin-12 (IL-12) und Interferon-alpha (IFN- ) zur Herstellung eines Medikaments zur Behandlung und Vorbeugung von Infektionen.

2. Verwendung nach Anspruch 1 wobei die Infektion eine chronische Infektion ist.

3. Verwendung nach Anspruch 2 wobei die Infektion eine chronische Virusinfektion ist.

4. Verwendung nach Anspruch 3 wobei die Infektion eine Hepatitis-, Papillom-, Humanimmundefizienz- oder eine Herpesvirusinfektion ist.

5. Verwendung nach Anspruch 1 oder 2 wobei die Infektion eine bakterielle Infektion ist.

6. Verwendung nach Anspruch 5 wobei die Infektion Tuberkulose, Salmonellose oder Listeriose ist.

7. Verwendung nach Anspruch 1 oder 2 wobei die Infektion eine parasitäre Infektion ist.

8. Verwendung nach Anspruch 7 wobei die Infektion Malaria, Leishmaniase oder Schistosomiase ist.

9. Interleukin-12 und Interferon- in einer synergistischen Kombination zur Behandlung von Krankheiten wie in einem der Ansprüche 1 bis 8 erwähnt.

10. Interleukin-12 und Interferon- in einer synergistischen Kombination mit einer oder mehreren weiteren Verbindungen zur Behandlung von Krankheiten wie in einem der Ansprüche 1 bis 8 erwähnt.

11. Pharmazeutische Zusammensetzung die eine synergistische Kombination von Interleukin-12 und Interferon- zusammen mit einem pharmazeutisch annehmbaren Träger beinhaltet.

12. Pharmazeutische Zusammensetzung nach Anspruch 11 welche eine oder mehrere weitere Verbindungen enthält die zur Behandlung von Infektionskrankheiten nützlich sind.

## Revendications

1. Utilisation d'une combinaison synergique d'interleukine-12 (IL-12) et d'interféron-alpha (IFN-α) pour la fabrication d'un médicament pour le traitement et la prévention des infections.

2. Utilisation selon la revendication 1, où l'infection est une infection chronique.

3. Utilisation selon la revendication 2, où l'infection est une infection chronique virale.

4. Utilisation selon la revendication 3, où l'infection est une hépatite, un papillome, l'immunodéficience humaine ou une infection par un herpès virus.

5. Utilisation selon la revendication 1 ou 2, où l'infection est une infection bactérienne.

6. Utilisation selon la revendication 5, où l'infection est une tuberculose, une salmonellose ou une listériose.

7. Utilisation selon la revendication 1 ou 2, où l'infection est une infection parasitaire.

8. Utilisation selon la revendication 7, où l'infection est le paludisme, la leishmaniose ou la schistosomiase.

9. Interleukine-12 et interféron-α dans une combinaison synergique pour le traitement des maladies citées dans une quelconque des revendications 1 à 8.

10. Interleukine-12 et interféron-α dans une combinaison synergique avec un ou plusieurs autres composés pour le traitement des maladies citées dans une quelconque des revendications 1 à 8.

11. Composition pharmaceutique contenant une combinaison synergique d'interleukine-12 et d'interféron-α avec un support pharmaceutiquement acceptable.

12. Composition pharmaceutique selon la revendication 11, contenant un ou plusieurs autres composés, utile pour le traitement de maladies infectieuses.
